Europäisches Patentamt

European Patent Office

Office européen des brevets

(11) Publication number: **0 553 814 A1**

## EUROPEAN PATENT APPLICATION

(21) Application number: **93101311.4**

(22) Date of filing: **28.01.93**

(51) Int. Cl.5: **C12P 23/00**

(30) Priority: **29.01.92 US 827774**

(43) Date of publication of application:
**04.08.93 Bulletin 93/31**

(84) Designated Contracting States:
**AT BE CH DE DK ES FR GB IT LI NL PT SE**

(71) Applicant: **PHILLIPS PETROLEUM COMPANY**
**5th and Keeler**
**Bartlesville Oklahoma 74004(US)**

(72) Inventor: **Nelles, Lynn P.**
**333 Stoneridge Court**
**Bartlesville, OK 74006(US)**
Inventor: **Wegner, Eugene Herman**
**618 Brookhollow Lane**
**Bartlesville, OK 74006(US)**

(74) Representative: **Dost, Wolfgang,**
**Dr.rer.nat.,Dipl.-Chem. et al**
**Patent- & Rechtsanwälte Bardehle .**
**Pagenberg . Dost . Altenburg . Frohwitter .**
**Geissler & Partner, Postfach 86 06 20**
**W-8000 München 86 (DE)**

(54) **Treatment of phaffia rhodozyma.**

(57) A process for enhancing the availability of astaxanthin in *Phaffia rhodozyma* yeast comprises the formation of an aqueous slurry of said *Phaffia rhodozyma* yeast in an aqueous solution of an acid selected from hydrochloric acid and sulfuric acid wherein said acid is present from 0.5 to 4 N, and thereafter heating the aqueous slurry at a temperature in the range of 60 to 90°C for 2 to 24 hours. The heated aqueous slurry is preferably further neutralized to pH 7 with a base selected from LiOH, NaOH, KOH, $Ca(OH)_2$ CaO, and $Mg(OH)_2$.

EP 0 553 814 A1

**Field of the Invention**

The invention relates to a process for treating *Phaffia rhodozyma* yeast.

**Background of the Invention**

Astaxanthin (trans-3,3'-dihydroxy-$\beta,\beta$-carotene-4,4'-dione) is an oxygenated carotenoid pigment used as a dietary pigment supplement for salmonids and crustaceans grown on aquacultural farms. One source of astaxanthin for the aquacultural use is the yeast *Phaffia rhodozyma*. However, regardless of the astaxanthin pigment level in the yeast cell, efficient uptake by the digestive tracts of fish and subsequent deposition in the flesh will not occur unless the cell walls are modified, making the internal, possibly membrane bound, pigment available. The *Phaffia rhodozyma* cell wall has a tough, capsular coating probably consisting mainly of mannans and $\alpha$-1,3-linked glucans (mutans) which cannot be broken down by most animals. It is necessary, therefore, to modify the yeast cell walls (without altering the astaxanthin) before incorporation into animal feeds.

Okagbue et al (Biotechnol. Letters, Vol. 5, pp. 731-736, 1983) has experimented with the cofermentation of *Phaffia rhodozyma* with *Bacillus circulans* WL-12. *B. circulans* WL-12 produces enzymes during cofermentation with *Phaffia rhodozyma* which partially digests the cell walls of *Phaffia rhodozyma*. Fleno et al (WIPO application 88/08025) discloses the use of mechanical means for breaking *Phaffia rhodozyma* cell walls, such as bead mills, french presses and valve homogenizers. Fleno et al also discloses a chemical extraction method utilizing glacial acetic acid to extract astaxanthin from *Phaffia rhodozyma* cells (requiring five separate immersions in glacial acetic acid at approximately 75°C).

Unfortunately, although these methods do work for small scaled preparations, for large scaled production of astaxanthin from *Phaffia rhodozyma*, these techniques are unsuitable. For example, cofermentation reduces the yield of astaxanthin, and is not a reliable means of producing astaxanthin in a form that would be readily available to salmonids and crustaceans. Mechanical breakage with bead mills has also proved ineffective, releasing only 40% of the available astaxanthin after three passes through a bead mill. Valve homogenizers have proven more effective as a mechanical means of breakage, being able to release 95% of the astaxanthin in three passes. However, valve homogenizers suitable for use in large scaled commercial operations are very expensive. Furthermore, having to perform three separate passes through a valve homogenizer will require extra storage tanks for recirculation and will be time consuming because of the low flow rates through the homogenizers.

Chemical extraction with glacial acetic acid is also impractical due to the large volumes of glacial acetic acid that would be necessary to extract the astaxanthin. Further, using this extraction method would require the astaxanthin to be recovered from the glacial acetic acid, and a recycle would have to be performed to recover the unused glacial acetic acid. Thus, it would be a significant contribution to the art to develop an inexpensive process for treating *Phaffia rhodozyma* cells, so that the astaxanthin contained therein will be readily available as a dietary pigment supplement for salmonids, crustaceans, and birds.

**Summary of the Invention**

It is therefore an object of the invention to provide a process to treat *Phaffia rhodozyma* yeast so that the astaxanthin present therein is made readily available as a dietary supplement to salmonids, crustaceans, and birds. It is also an object of the invention to provide an inexpensive process for extracting astaxanthin from the *Phaffia rhodozyma* yeast. Other objects, advantages, features and aspects of the invention will become more apparent hereinafter as the invention is more fully described in the following disclosure and claims.

According to the present invention, we have discovered a process for enhancing the availability of astaxanthin in *Phaffia rhodozyma* yeast which comprises contacting *Phaffia rhodozyma* yeast with an aqueous solution of an acid selected from the group consisting of hydrochloric acid and sulfuric acid thereby forming an aqueous slurry wherein the acid is present in an effective amount to modify the yeast's cell walls; and thereafter heating the aqueous slurry at a temperature in the range of from about 60°C to about 90°C for about 2 hours to about 24 hours.

Detailed Description of the invention

*Phaffia rhodozyma* cells containing astaxanthin should be treated with an effective amount of an acid selected from the group consisting of hydrochloric and sulfuric acid to provide the astaxanthin available as a dietary pigment supplement for salmonids, crustaceans and birds.

The acid should be employed in an aqueous solution which will be admixed with the *Phaffia rhodozyma* cells to form an aqueous slurry. The amount of the acid used should be enough to obtain a final acid concentration of the aqueous slurry in the range of from about 0.5 N to about 4 N. Preferably the acid concentration in the aqueous slurry will range from about 0.75 N to about 1 N. The preferred acid for the practice of this invention is hydrochloric acid.

Generally speaking, the present invention may be used on any strain of *Phaffia rhodozyma*. However, it is preferred that the method of the present invention be used on *Phaffia rhodozyma* cells which have been grown in an exponential growth phase because these cells tend to have weaker cell walls. The concentration of *Phaffia rhodozyma* cells utilized in the present invention does not appear to be particularly important. The availability of astaxanthin (which may be referred to as breakage) is independent of cell concentration over a wide range of concentration from in the range of from about 42 grams/liter on a dry weight basis to about 144 grams/liter on a dry weight basis.

Admixing the acid and *Phaffia rhodozyma* cells may be accomplished by any suitable means with minimum stirring to avoid volatilizing the acid component. Additionally, it is desirable to also utilize a closed container to further enhance the effectiveness of cell lysis by acid treatment. The aqueous slurry can also be formed by adding the acid directly to the fermentor broth containing the *Phaffia rhodozyma* cells. Alternatively, a wash cake of *Phaffia rhodozyma* cells can be resuspended in an aqueous solution prior to the addition of the acid.

After the aqueous slurry containing the acid has been formed, it should be heated to a temperature of in the range of from about 60°C to about 90°C and, preferably, about 80°C. The increased temperature accelerates the rate of cell wall breakage. At these elevated temperatures, substantially all the astaxanthin contained within the *Phaffia rhodozyma* cells will be available within from about 2 hours to about 24 hours and, generally, it will be available in from about 3 hours to about 6 hours. Generally speaking, higher acid concentrations and higher temperatures will result in the astaxanthin becoming available much quicker. However, as demonstrated in Example IV, a concentration of from about 0.75 N to about 1 N hydrochloric acid in a closed container heated to about 80°C will provide a high availability of astaxanthin in only 3 hours.

After the *Phaffia rhodozyma* cells have been treated with the acid and heated, the aqueous slurry may be neutralized with a base to about pH 7. Suitable bases are selected from the group consisting of LiOH, NaOH, KOH, $Ca(OH)_2$, CaO, and $Mg(OH)_2$.

Further processing of the aqueous slurry to recover the astaxanthin may also be performed including but not limited to spray drying, drum drying, freeze drying, and other suitable methods.

The following examples are provided to further illustrate the practice of the present invention.

Example I

Sulfuric acid was added, to final concentrations shown in Table I below, to *Phaffia rhodozyma* cell broth (6 ml), containing about 42 g/l based on dry cell weight, in closed glass tubes for treatment at room temperature and in closed polyethylene bottles for treatments at higher temperatures. A series of samples were then incubated for 16 hours at temperatures indicated in Table I. The acid-treated cells were then examined for percent breakage as shown in Table I.

## Table I

### Sulfuric Acid Lysis of *Phaffia rhodozyma* Yeast

| Run No. | Acid Conc'n (N) | Cell Breakage[a] | | |
| --- | --- | --- | --- | --- |
| | | Room Temp. | 37°C | 60°C |
| 1 | 0.6 | -[b] | - | 16 |
| 2 | 1.0 | - | - | 54 |
| 3 | 2.0 | 7 | 9 | 91 |
| 4 | 4.0 | 8 | 11 | 96 |
| 5 | 6.0 | 32 | 41 | 90 |

[a]Acetone (0.975 ml) was added to 2 microcentrifuge tubes containing an aliquot (0.025 ml) of *Phaffia rhodozyma* yeast broth. Glass beads (0.25 grams, 450-500 microns) were added to one sample and both tubes were vortexed on a Vortex Jr. Mixer (Scientific Products, Inc.) for a total of 10 minutes at 4°C. Glass beads and cell debris were pelleted by centrifugation at 1,000 x g for 5 minutes. A 0.5 ml aliquot of the supernatant was removed and added to 1.0 ml of acetone. Absorbance was measured on 478 nm. Percent cell breakage was determined by calculating the ratio:

$$\frac{A_{478} \text{ (no glass beads)}}{A_{478} \text{ (glass beads)}} * 100\%$$

[b]The breakage was verified microscopically.
- indicates runs were not done.

Table I indicates that sulfuric acid aids in *Phaffia rhodozyma* cell breakage and the higher the temperature (runs 3 to 5), the more effective the acid is in lyzing the yeast cells.

Example II

This example demonstrates that not all acids can affect the cell lysis of *Phaffia rhodozyma* yeast.

A series of runs were carried out the same as those described in Example I except that sulfuric acid was replaced with the acids indicated in Table II below, the acid concentration was 1 N, and the incubation was at 60°C for 24 hours.

### Table II

| *Phaffia rhodozyma* Cell Lysis with different acids | | |
| --- | --- | --- |
| Run No. | Acid[a] | % Breakage[b] |
| 6 | $H_2SO_4$ | 60 |
| 7 | $H_2SO_4 + H_3PO_4$ | 14 |
| 8 | $H_3PO_4$ | 18 |
| 9 | $H_2SO_4 + HCl$ | 93 |
| 10 | HCl | 93 |
| 11 | $H_3PO_4 + HCl$ | 18 |
| 12 | $CH_3COOH^c$ | 13 |

[a]Total concentration was 1N in all runs; mixtures were equi-normal.
[b]See footnote a, Table I.
[c]The incubation was 16 hours for acetic acid treatment.

Table II demonstrates that some acids, such as $H_3PO_4$ and acetic acid (runs 8 and 12), were not effective in lyzing *Phaffia rhodozyma* yeast cells. It also shows that $H_3PO_4$, when used in combination with either $H_2SO_4$ (run 7) or HCl (run 11), unexpectedly reduced the effectiveness of $H_2SO_4$ or HCl. It was further demonstrated that the effect of HCl on cell lysis was not due to chloride ions because treatment of the cells with NaCl under same conditions did not cause any cell breakage.

### Example III

This example demonstrates that an acid-promoted cell lysis of *Phaffia rhodozyma* is insensitive to cell density.

A series of runs were carried out the same as those described in Example I except that 1 N HCl was used, the incubation was at 80 °C for 4 hours, and the cell density in the broth varied from 48 g/l to 144 g/l. The results of cell breakage are shown in Table III.

Table III

| Acid-promoted *Phaffia rhodozyma* Cell Lysis at High Cell Densities | | |
|---|---|---|
| Run No. | Cell Density (g/l)[a] | % Breakage[b] |
| 13 | 48 | 90 |
| 14 | 96 | 92 |
| 15 | 144 | 90 |

[a]Cell density was calculated g dry cell weight per liter.
[b]See footnote a, Table I.

The results shown in Table III indicate that the invention is well adapted to yeast broth containing high cell densities.

### Example IV

This example shows that the acid-promoted cell lysis becomes ineffective if it is carried out in an open container.

The runs were carried out the same as in Example I except that the concentration of HCl used was 0.75 N and the cell lysis was carried out in an open container. The results obtained after 4 hours incubation indicated that 75% of cells in closed container (control) were broken whereas no cells were broken in the open container. The results further show that there was no volatilization of HCl because both sealed and non-sealed containers required the same amounts of base to neutralize.

### Example V

This example illustrates that acid treatment at mild condition facilitates the mechanical rupturing of the yeast cells.

A series of runs were carried out the same as those shown in Example I except that cells were incubated in HCl at the concentrations shown in Table IV, at 60 °C for 16 hours and thereafter the cells were further broken by passage through Microfluidics, a cell homogenizer. The results are shown in Table IV.

Table IV

| Phaffia rhodozyma Cell Breakage | | | |
|---|---|---|---|
| Run No. | Acid Conc'n (N) | Microfluidics | % Breakage |
| 16 | 0 | No | 0 |
| 17 | 0.6 | No | 8 |
| 18 | 1.0 | No | 69 |
| 19 | 0 | 1 pass | 36 |
| 20 | 0.6 | 1 pass | 38 |
| 21 | 1.0 | 1 pass | 96 |

The results demonstrate that acid treatment of *Phaffia rhodozyma* cells greatly increased the % cell breakage by a cell homogenizer. For example, at 1.0 N, the breakage increased from 36 (run 19) to 96% (run 21).

A variety of acids were experimented with to determine the most effective acids for breaking *Phaffia rhodozyma's* cell walls. Initially, acetic acid, formic acid, sulfuric acid, phosphoric acid and hydrochloric acid were screened. As is demonstrated in Table II, only sulfuric acid and hydrochloric acid are effective in breaking *Phaffia rhodozyma's* cell wall thereby making the astaxanthin contained within the cells available.

The examples have been provided merely to illustrate the practice of the invention and should not be read so as to limit the scope of the invention or the appended claims in any way.

Reasonable variations and modifications, not departing from the essence and spirit of the invention, are contemplated to be within the scope of patent protection desired and sought.

**Claims**

1. A process for enhancing the availability of astaxanthin in *Phaffia rhodozyma* yeast characterized by forming an aqueous slurry of said *Phaffia rhodozyma* yeast in an aqueous solution of an acid selected from hydrochloric acid and sulfuric acid and heating the aqueous slurry at a temperature in the range of 60 to 90°C for a time period from 2 to 24 hours.

2. The process according of claim 1 wherein said temperature is maintained at about 80°C.

3. The process of claim 1 or 2 wherein said aqueous slurry has an acid concentration from 0.5 to 4 N.

4. The process of claim 3 wherein said aqueous slurry has an acid concentration from 0.75 to 1 N.

5. The process of any of the preceding claims wherein said *Phaffia rhodozyma* cells are used in an amount to result in a concentration from 42 to 144 g/l on a dry weight basis.

6. The process of any of the preceding claims wherein said aqueous slurry is heated for a time period of 3 to 6 hours.

7. The process of any of the preceding claims wherein said heated aqueous slurry is further neutralized to about pH 7 with a base selected from LiOH, NaOH, KOH, Ca(OH)$_2$ CaO, and Mg(OH)$_2$.

8. The process of any of the preceding claims wherein said heating is carried out in a closed container.

## DOCUMENTS CONSIDERED TO BE RELEVANT

EP 93101311.4

| Category | Citation of document with indication, where appropriate, of relevant passages | Relevant to claim | CLASSIFICATION OF THE APPLICATION (Int. Cl.5) |
|---|---|---|---|
| A | EP - A - 0 454 024 (PHILLIPS PETROLEUM COMPANY) * Claims 7,8 * | 1 | C 12 P 23/00 |
| D,A | WO - A - 88/08 025 (DANISCO BIOTEKNOLOGI) * Claims 9-20 * | 1 | |
| A | EP - A - 0 427 405 (ENZYMATIK) * Abstract * | 1 | |
| A | EP - A - 0 438 182 (PHILLIPS PETROLEUM COMPANY) * Claims 8-11 * | 1 | |

TECHNICAL FIELDS SEARCHED (Int. Cl.5)

C 12 P

The present search report has been drawn up for all claims

| Place of search | Date of completion of the search | Examiner |
|---|---|---|
| VIENNA | 02-04-1993 | WOLF |